# EUROPÄISCHE PATENTSCHRIFT

(11) **EP 4 599 877 B1**
(45) Veröffentlichungstag und Bekanntmachung des Hinweises auf die Patenterteilung: **04.11.2026**
(21) Anmeldenummer: 25155474.7
(22) Anmeldetag: 03.02.2025
(51) Int. Cl.: A61M 25/00, A61B 5/00

(54) **KATHETER ZUR VERWENDUNG BEI EINER SCHMERZTHERAPIE**
CATHETER FOR USE IN PAIN THERAPY
CATHÉTER DESTINÉ À ÊTRE UTILISÉ DANS UNE THÉRAPIE DE DOULEUR

(30) Priorität: 06.02.2024 DE 102024103223
(43) Veröffentlichungstag der Anmeldung: 13.08.2025
(73) Patentinhaber: B. Braun Melsungen AG, 34212 Melsungen (DE)
(72) Erfinder: Wildhagen, Jens, 30659 Hannover (DE)
(74) Vertreter: Patentanwälte Ruff, Wilhelm, Beier, Dauster & Partner mbB

(56) Entgegenhaltungen:
- US-A- 5 968 012
- US-A1- 2004 143 240

## Beschreibung

Die Erfindung betrifft einen Katheter zur Verwendung bei einer Schmerztherapie, aufweisend einen Katheterschaft, der zwischen einem proximalen Ende und einem distalen Ende längserstreckt ist, eine Katheterspitze, die an dem distalen Ende angeordnet ist, und einen Katheteransatz, der an dem proximalen Ende angeordnet ist.

Derartige Katheter sind im Bereich der Medizintechnik allgemein bekannt und zur Verwendung bei einer Schmerztherapie vorgesehen. Bei einer als peripherer Nervenblock (PNB) bezeichneten Schmerztherapie wird der Katheter unter Zuhilfenahme einer Einführhilfe so weit distal vorgeschoben, bis die Katheterspitze unmittelbar an dem zu betäubenden Nerv platziert ist. Als Einführhilfe werden Kanülen und/oder Kapillare verwendet, wobei prinzipiell zwischen unterschiedlichen Anlagetechniken unterschieden werden kann ("Kanüle über die Nadel", "Kanüle durch die Nadel", "Kanüle durch das Kapillar"). Bei manchen Schmerztherapien wird der Katheter zur Verabreichung eines Lokalanästhetikums verwendet. Zur Betäubung des Nervs wird das Lokalanästhetikum über einen oder mehrere im Bereich der Katheterspitze angeordnete Katheterauslässe abgegeben. Bei anderen Schmerztherapien wird der Katheter zur elektrischen Neurostimulation verwendet. Die elektrische Neurostimulation (kurz: Neurostimulation oder Neuromodulation) sieht die Abgabe von Stromimpulsen in unmittelbarer Nähe eines Nervs vor, um dessen Leitungsverhalten für Schmerzimpulse zu beeinflussen. Die EP 3274036 offenbart einen Katheter zur Verabreichung von Anästhetika an eine periphere Nervenstelle eines Patienten, insbesondere einen Katheter mit einem aufblasbaren Ballon, der am Ende des Katheters angeordnet ist und bei der Anwendung an der peripheren Nervenstelle platziert wird.

Aufgabe der Erfindung ist es, einen Katheter der eingangs genannten Art bereitzustellen, der eine verbesserte Schmerztherapie ermöglicht.

Diese Aufgabe wird dadurch gelöst, dass der Katheterschaft einen Vorspannabschnitt aufweist, der zwischen einem Vorspannzustand und einem Ausgleichszustand elastisch deformierbar ist. In dem Vorspannzustand ist der Vorspannabschnitt axial elastisch gestaucht und weist eine erste Länge auf. In dem Ausgleichszustand ist der Vorspannabschnitt nicht oder weniger stark axial gestaucht und weist eine größere zweite Länge auf. Zwischen dem Vorspannzustand und dem Ausgleichszustand führt der Vorspannabschnitt eine durch die elastische Vorspannung bewirkte Ausgleichsbewegung aus, mittels derer bei einer proximalen Dislokation des Katheteransatzes eine proximale Dislokation der Katheterspitze vermieden und/oder ausgeglichen werden kann. Die Erfindung geht von der Erkenntnis aus, dass bei Verwendung des Katheters zur Verabreichung eines Lokalanästhetikums ein unbeabsichtigtes Zurückziehen der Katheterspitze (proximale Dislokation) zu einer nicht ausreichenden Umspülung des Nervs mit dem Lokalanästhetikum führen kann. Bei Verwendung des Katheters zur Neurostimulation kann eine proximale Dislokation zu einer suboptimalen Stimulation des Nervs führen. Bei beiden Behandlungsmethoden kann hierdurch eine Wirksamkeit der Schmerztherapie beeinträchtigt sein. Durch die erfindungsgemäße Lösung werden solche proximalen Dislokationen der Katheterspitze vermieden. Zu diesem Zweck weist der Katheterschaft den besagten Vorspannabschnitt auf. Der Vorspannabschnitt lässt sich durch Ausüben einer äußeren axialen Druckbelastung stauchen. Dabei verkürzt sich die axiale Länge des Vorspannabschnitts und damit gleichzeitig auch eine axiale Länge des Katheterschafts. Dieser Zustand wird als Vorspannzustand bezeichnet. In diesem weist der Vorspannabschnitt die besagte (gestauchte) erste Länge auf. Im Vorspannzustand übt der Vorspannabschnitt eine Vorspannung auf das distale Ende und das proximale Ende aus, so dass die beiden Enden axial voneinander weg vorgespannt sind. Der Vorspannabschnitt wird so weit gestaucht, bis sich dessen elastische Vorspannung und die äußere Druckbelastung ausgleichen. Unter Druckbelastung wird eine axiale Kraftausübung auf den Vorspannabschnitt von beiden Seiten des Vorspannabschnitts axial aufeinander zu verstanden. Sobald die Druckbelastung verringert oder vollständig entfernt wird, streckt sich der Vorspannabschnitt elastisch bis wieder ein Gleichgewicht zwischen äußerer Druckbelastung und Vorspannung vorliegt. Dieser Zustand wird als Ausgleichszustand bezeichnet. Im Ausgleichszustand weist der Vorspannabschnitt die besagte axiale zweite Länge auf, die größer als die erste Länge ist. Durch die elastische Ausgleichsbewegung des Vorspannabschnitts beim Übergang vom Vorspannzustand in den Ausgleichszustand gleicht der Vorspannabschnitt eine proximale Dislokation des Katheteransatzes aus, so dass keine oder zumindest eine geringere proximale Dislokation der Katheterspitze erfolgt.

Bei der erfindungsgemäßen Lösung bewirkt die elastische Deformierbarkeit des Vorspannabschnitts, dass bei Wegnahme oder Verringerung der Druckbelastung der Vorspannabschnitt von selbst, d.h. ohne weitere äußere Krafteinwirkung neben der Druckentlastung, selbsttätig vom Vorspannzustand in den Ausgleichszustand deformiert. Dadurch ist insbesondere keine Zugkraft notwendig, die an dem Vorspannabschnitt oder an dem proximalen Ende oder dem Katheteransatz angreift und die axiale Längung des Vorspannabschnitts hervorruft. Bei vorteilhaften Ausführungsformen ist die elastische Deformierbarkeit eine rein elastische Deformierbarkeit ohne praktisch erhebliche plastische oder elastisch-plastische Deformationsanteile. Der erfindungsgemäße Katheter wird derart am Körper angelegt, dass die Katheterspitze im Körpergewebe nahe einem zu betäubenden Nerv angeordnet ist und der Vorspannabschnitt im Vorspannzustand vorliegt. Der Vorspannabschnitt spannt die Katheterspitze in distaler Richtung vor, so dass die Katheterspitze axial gegen das Körpergewebe drückt. Das Körpergewebe übt eine axiale Reaktionskraft (Druckbelastung) auf die Katheterspitze aus und hält dadurch den Vorspannabschnitt im Vorspannzustand. Bei einer proximalen Dislokation des Katheteransatzes bewegt sich zunächst auch die Katheterspitze proximal um eine infinitesimale Weglänge. Hierdurch verringert sich die axiale Reaktionskraft. Dadurch übersteigt die distale Vorspannung, die der Vorspannabschnitt auf die Katheterspitze in distaler Richtung ausübt, die axiale entgegengerichtete Druckbelastung. Infolgedessen streckt sich der Vorspannabschnitt, bis ein Kräftegleichgewicht zwischen Vorspannkraft und Druckbelastung vorliegt. Die Längenzunahme des Katheterschafts im Bereich des Vorspannabschnitts bewirkt eine entsprechend verringerte und im besten Fall keine proximale Dislokation der Katheterspitze. Der Vorspannabschnitt kann auch als federelastisch deformierbar, elastisch komprimierbar und/oder elastisch stauchbar bezeichnet werden. Die Längenzunahme des Vorspannabschnitts kann auch als Elongation bezeichnet werden.

In Ausgestaltung der Erfindung ist der Vorspannabschnitt aus einem elastisch stauchbaren Material gefertigt. Vorzugsweise ist das elastisch stauchbare Material ein Elastomermaterial. Bei dieser Ausgestaltung der Erfindung ist der Katheterschaft folglich wenigstens im Bereich des Vorspannabschnitts aus dem besagten elastisch stauchbaren Material gefertigt, wobei der Katheterschaft abseits des Vorspannabschnitts aus einem weniger leicht stauchbaren Material gefertigt ist. Sofern der Vorspannabschnitt die gesamte Länge des Katheterschafts einnimmt, ist folglich der gesamte Katheterschaft aus dem besagten elastisch stauchbaren Material gefertigt.

In weiterer Ausgestaltung der Erfindung weist der Vorspannabschnitt eine Feder auf. Vorzugsweise ist die Feder eine Schraubenfeder.

In weiterer Ausgestaltung der Erfindung weist der Vorspannabschnitt eine elastisch stauchbare Gestaltgebung auf. Bei dieser Ausgestaltung der Erfindung wird die elastische Deformierbarkeit des Vorspannabschnitts nicht unbedingt durch eine geeignete Materialwahl, sondern durch eine entsprechende Gestaltgebung erreicht. Die Längenzunahme oder Längenverringerung erfolgt aufgrund einer Gestaltänderung des Vorspannabschnitts, beispielsweise aufgrund einer Ziehharmonikabewegung. Bei dieser Ausgestaltung der Erfindung weist der Katheterschaft folglich wenigstens im Bereich des Vorspannabschnitts die besagte elastisch stauchbare Gestaltgebung auf, wobei der Katheterschaft abseits des Vorspannabschnitts eine unterschiedliche und weniger leicht stauchbare Gestaltgebung aufweist, vorzugsweise eine aus dem Stand der Technik bekannte glattwandige zylindrische Gestaltung. Sofern der Vorspannabschnitt die gesamte Länge des Katheterschafts einnimmt, weist folglich der gesamte Katheterschaft die besagte elastisch stauchbare Gestaltgebung auf.

In weiterer Ausgestaltung der Erfindung ist die elastisch stauchbare Gestaltgebung eine ziehharmonikaartige Gestaltung. Bei dieser Ausgestaltung ist der Vorspannabschnitt nach Art einer Ziehharmonika gestaltet. Hierdurch kann der Vorspannabschnitt bei einer entsprechenden Druckbelastung leicht gestaucht werden. Die ziehharmonikaartige Gestaltung kann insbesondere durch eine gefaltete oder wellenförmige oder wendelförmige Ausbildung des Vorspannabschnitts gebildet sein.

In weiterer Ausgestaltung der Erfindung ist der Vorspannabschnitt über eine gesamte Länge des Katheterschafts längserstreckt. Folglich ist bei dieser Ausgestaltung der Erfindung der gesamte Katheterschaft elastisch stauchbar. Diese Ausgestaltung der Erfindung erlaubt eine vereinfachte Herstellung, da der Katheterschaft aus ein- und demselben Material gefertigt sein kann und/oder ein- und dieselbe Gestaltgebung aufweisen kann. Dies ist im Unterschied zu Ausführungsformen, bei denen der Vorspannabschnitt lediglich einen Teil der Gesamtlänge des Katheterschafts einnimmt.

In einer weiteren anderen Ausgestaltung der Erfindung ist der Vorspannabschnitt über lediglich einen Teil der Gesamtlänge des Katheterschafts längserstreckt und der Katheterschaft weist abseits des Vorspannabschnitts eine Drucknachgiebigkeit auf, die geringer ist als eine Drucknachgiebigkeit des Vorspannabschnitts. Mit Nachgiebigkeit ist das Reziproke der Steifigkeit gemeint. Beispielsweise kann der Katheterschaft abseits des Vorspannabschnitts eine Drucknachgiebigkeit aufweisen, die maximal 20 %, bevorzugt maximal 5 %, besonders bevorzugt maximal 1 %, der Drucknachgiebigkeit des Vorspannabschnitts beträgt. Hierdurch deformiert sich der Vorspannabschnitt beim Ausüben einer Druckbelastung auf den Katheterschaft elastisch, während sich der Katheterschaft abseits des Vorspannabschnitts weniger oder sogar gar nicht elastisch verformt.

In weiterer Ausgestaltung der Erfindung weist der Katheterschaft abseits des Vorspannabschnitts eine unterschiedliche Gestaltgebung auf. Durch die unterschiedlichen Gestaltgebungen des Vorspannabschnitts und des Katheterschafts abseits des Vorspannabschnitts kann der Vorspannabschnitt eine Drucknachgiebigkeit aufweisen, die größer ist als eine Drucknachgiebigkeit des Katheterschafts abseits des Vorspannabschnitts.

In weiterer Ausgestaltung der Erfindung weist der Katheter eine Längenbegrenzungseinrichtung auf, die zur Begrenzung der zweiten Länge des Vorspannabschnitts eingerichtet ist. Die Längenbegrenzungseinrichtung begrenzt die zweite Länge des Vorspannabschnitts auf einen vorbestimmten Wert, die als Begrenzungslänge bezeichnet werden kann. Die Längenbegrenzungseinrichtung verhindert eine Streckung des Vorspannabschnitts über die Begrenzungslänge hinaus. Denkbar ist auch, dass mittels der Längenbegrenzungseinrichtung eine Stauchung des Vorspannabschnitts möglich ist.

In weiterer Ausgestaltung der Erfindung weist die Längenbegrenzungseinrichtung ein längserstrecktes Zugelement auf, das den Vorspannabschnitt unter Ausbildung einer Kraftentlastung überbrückt. Das Zugelement bewirkt eine mechanische Überbrückung des Vorspannabschnitts, sobald dieser eine maximal zulässige Länge erreicht. Bei Erreichen der maximal zulässigen Länge nimmt das Zugelement die durch den Vorspannabschnitt aufgebrachte Druckkraft auf und bildet dadurch die besagte Kraftentlastung aus, die auch als Druckentlastung bezeichnet werden kann . Das Zugelement kann auf jede für den vorliegenden Zweck geeignete Weise gestaltet sein. Bei manchen Ausführungsformen ist das Zugelement auf beiden axialen Seiten des Vorspannabschnitts befestigt, beispielsweise an den axialen Enden des Vorspannabschnitts und/oder am distalen Ende und/oder am proximalen Ende des Katheterschafts.

In weiterer Ausgestaltung der Erfindung weist das längserstreckte Zugelement einen elektrisch leitfähigen Draht auf, der zur Übertragung und/oder Abgabe von Stromimpulsen zur Neurostimulation eingerichtet ist. Der Draht weist folglich eine vorteilhafte Mehrfachfunktion auf. Zum einen fungiert der Draht als Kraftentlastung. Zum anderen fungiert der Draht zusätzlich als elektrischer Leiter für die besagte Neurostimulation. Bei dieser Ausgestaltung der Erfindung weist der Katheterschaft und/oder die Katheterspitze wenigstens eine Stimulationselektrode auf, die mittels des leitfähigen Drahts elektrisch leitfähig kontaktiert oder durch den Draht gebildet ist.

In weiterer Ausgestaltung der Erfindung weist der Katheter eine Halteeinrichtung auf, die dazu eingerichtet ist, den Vorspannabschnitt im Vorspannzustand gegen eine Deformation zu sichern und bei einer Aktivierung eine Deformation in Richtung des Ausgleichzustands freizugeben. Durch die Aktivierung kann der Zeitpunkt bestimmt werden, in dem eine mögliche Elongation des Vorspannabschnitts zugelassen oder freigegeben ist. Beispielsweise kann die Halteeinrichtung bei der Anlage des Katheters den Vorspannabschnitt im Vorspannzustand fixieren. Hierdurch wird vermieden, dass der Vorspannabschnitt bereits vor endgültiger Positionierung der Katheterspitze nahe dem zu betäubenden Nerv in den Ausgleichszustand übergeht. Insbesondere kann ein Anlegen des Katheters derart erfolgen, dass der Katheter mit der Katheterspitze in Richtung eines Nervs vorgeschoben wird, während die Halteeinrichtung den Vorspannabschnitt im Vorspannzustand hält und dessen Deformation verhindert. Nach Erreichen der endgültigen Position der Katheterspitze kann die Halteeinrichtung aktiviert werden, wodurch eine Deformation des Vorspannabschnitts freigegeben und erst dann möglich ist. Bei manchen Ausgestaltungen kann es möglich sein, die Halteeinrichtung nach einer Aktivierung wieder zu deaktivieren, so dass der Vorspannabschnitt in dem Zustand, in dem dieser sich bei Deaktivierung der Halteeinrichtung befindet, gehalten wird, so dass keine weitere Streckung und/oder Stauchung des Vorspannabschnitts erfolgen kann. Bei manchen Ausführungsformen ist die Längenbegrenzungseinrichtung und die Halteeinrichtung durch ein und dieselbe Komponente gebildet, die beide Funktionen erfüllt. Die Aktivierung kann auch als Betätigung bezeichnet werden.

Bei manchen Ausgestaltungen liegt der Vorspannabschnitt bei der Anlage des Katheters im Ausgleichszustand vor. Das beim Vorschieben der Katheterspitze zum zu betäubenden Nerv zu durchdringende Körpergewebe übt eine axiale Druckkraft auf die Katheterspitze aus, so dass der Vorspannabschnitt gestaucht wird. Sobald die Katheterspitze in der Nähe des Nervs angeordnet ist, kann dann der Vorspannabschnitt im Vorspannzustand vorliegen.

In weiterer Ausgestaltung der Erfindung weist der Katheter eine Stützstruktur auf, die den Vorspannabschnitt gegen eine Knickbewegung in einer Richtung quer zur Längserstreckung sichert. Die Stützstruktur kann dafür sorgen, dass der Vorspannabschnitt seine elastische Deformierbarkeit zuverlässig beibehält, vorzugsweise indem die Stützstruktur den Vorspannabschnitt radial stabilisiert. In vorteilhaften Ausführungen umgibt die Stützstruktur den Vorspannabschnitt in Umfangsrichtung vollständig. Bei manchen Ausgestaltungen erstreckt sich die Stützstruktur über einen Teil des Vorspannabschnitts, so dass ein anderer Teil des Vorspannabschnitts nicht von der Stützstruktur umgeben ist. Bei anderen Ausgestaltungen erstreckt sich die Stützstruktur über die gesamte Länge des Vorspannabschnitts oder sogar axial über den Vorspannabschnitt hinaus und über einen abseits des Vorspannabschnitts gelegenen Teil des Katheterschafts.

In weiterer Ausgestaltung der Erfindung weist der Katheterschaft ein Katheterlumen zur Verabreichung eines Lokalanästhetikums auf. Hierdurch kann das Lokalanästhetikum direkt über den Katheter an das umgebende Körpergewebe abgegeben werden. Vorzugsweise ist wenigstens ein Katheterauslass im Bereich der Katheterspitze vorhanden, über den das Lokalanästhetikum an das Körpergewebe abgegeben werden kann. Bei manchen Ausführungsformen erstreckt sich das Katheterlumen durch den Vorspannabschnitt hindurch, während bei anderen Ausführungsformen das Katheterlumen am Vorspannabschnitt vorbei erstreckt ist.

Die Erfindung betrifft zudem eine Katheteranordnung mit einem Katheter gemäß der vorhergehenden Beschreibung und mit einer Einführhilfe, die zwischen einem distalen Ende und einem proximalen Ende längserstreckt ist und ein Einführlumen zum Einführen des Katheterschafts aufweist. Die Einführhilfe kann auch als Kapillar bezeichnet werden. Bei dieser Ausgestaltung braucht der Katheter selbst kein Katheterlumen aufzuweisen, um ein Lokalanästhetikum verabreichen zu können.

In weiterer Ausgestaltung der Erfindung fungiert bei in das Einführlumen eingeführtem Katheter die Einführhilfe als Stützstruktur für den Vorspannabschnitt. Hierdurch erfüllt die Einführhilfe eine Mehrfachfunktion. Zum einen unterstützt die Einführhilfe ein Anlegen des Katheters. Zum anderen sichert die Einführhilfe den Vorspannabschnitt gegen eine Knickbewegung in einer Richtung quer zur Längserstreckung.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in schematischer Seitenansicht eine Ausführungsform eines erfindungsgemäßen Katheters mit einem Katheterschaft, der einen Vorspannabschnitt in einem Vorspannzustand aufweist,
- Fig. 2: eine weitere schematische Seitenansicht des Katheters nach Fig. 1, wobei der Vorspannabschnitt in einem Ausgleichszustand vorliegt,
- Fig. 3, 4: eine schematische Seitenansicht einer Ausführungsform eines Vorspannabschnitts mit elastisch stauchbarer Gestaltgebung in einem gestauchten Zustand (Fig. 3) und in einem nicht gestauchten Zustand (Fig. 4),
- Fig. 5: eine schematische Seitenansicht einer weiteren Ausführungsform eines erfindungsgemäßen Katheters, wobei der Vorspannabschnitt über eine gesamte Länge des Katheterschafts erstreckt ist,
- Fig. 6: eine schematische Seitenansicht einer Katheteranordnung mit einem Katheter mit einem Vorspannabschnitt, der eine Feder aufweist, und einer Einführhilfe, wobei die Einführhilfe in einem Längsschnitt dargestellt ist, und
- Fig. 7: eine schematische Seitenansicht einer weiteren Ausführungsform eines erfindungsgemäßen Katheters mit einer Längenbegrenzungseinrichtung und einer Halteeinrichtung.

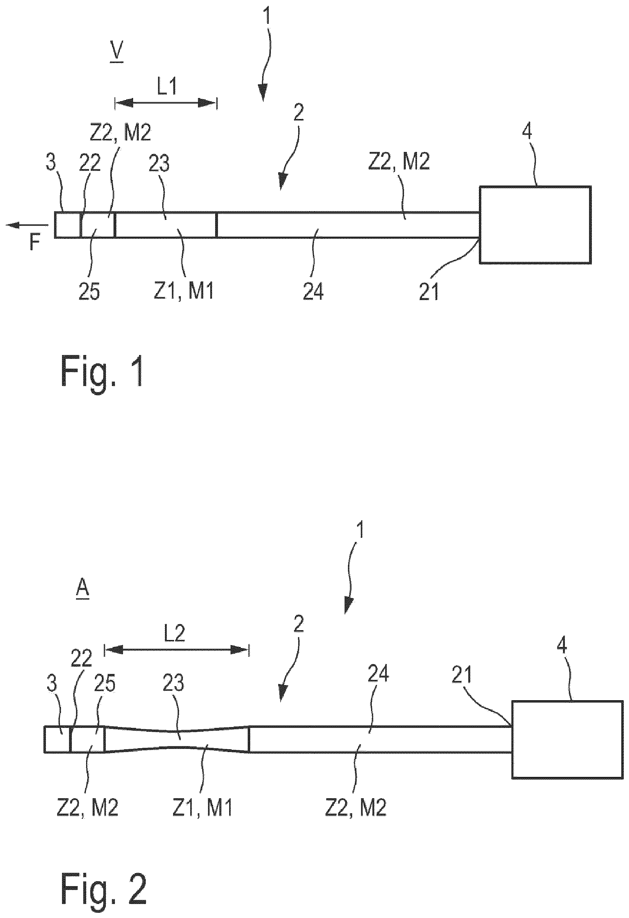

Gemäß Fig. 1 ist ein Katheter 1 zur Verwendung bei einer Schmerztherapie vorgesehen, die auch als peripherer Nervenblock bezeichnet wird. Der Katheter 1 kann auch als Schmerzkatheter bezeichnet werden.

Der Katheter 1 weist einen Katheterschaft 2, eine Katheterspitze 3 und einen Katheteransatz 4 auf.

Der Katheterschaft 2 ist zwischen einem proximalen Ende 21 und einem distalen Ende 22 längserstreckt.

Die Katheterspitze 3 ist an dem distalen Ende 22 des Katheterschafts 2 angeordnet. Die Katheterspitze 3 ist bei der gezeigten Ausführungsform gesondert von dem Katheterschaft 2 gefertigt und fest mit dessen distalem Ende 22 verbunden. Alternativ kann die Katheterspitze 3 ein integraler Abschnitt des Katheterschafts 2 sein und dessen distales Ende 22 bilden.

Der Katheteransatz 4 ist an dem proximalen Ende 21 des Katheterschafts 2 angeordnet. Der Katheteransatz 4 ist fest mit dem proximalen Ende 21 verbunden. Der Katheteransatz 4 kann auch als Katheter-Hub (englisch: catheter hub) bezeichnet werden.

Die Schmerztherapie kann eine Verabreichung eines Lokalanästhetikums umfassen. Es versteht sich, dass in diesem Fall der Katheter 1 wenigstens ein durch den Katheterschaft 2 und den Katheteransatz 4 längserstrecktes Katheterlumen und wenigstens einen fluidleitend mit dem Katheterlumen verbundenen Katheterauslass zur Abgabe des Lokalanästhetikums aufweisen kann. Der wenigstens eine Katheterauslass und das Katheterlumen sind vorliegend nicht im Detail gezeigt. Der Katheterauslass oder die Katheterauslässe sind vorzugsweise im Bereich der Katheterspitze 3 angeordnet. Dabei kann der wenigstens eine Katheterauslass axial durch die Katheterspitze 3 und/oder radial durch den Katheterschaft 2 erstreckt sein. Die Anzahl und Anordnung der Katheterauslässe ist im Hinblick auf die vorliegende Erfindung nicht wesentlich, so dass weitere Details nicht erläutert werden. Bei anderen Ausführungsformen dient der Katheter 1 zur elektrischen Neurostimulation. Zu diesem Zweck kann der Katheter 1 eine oder mehrere Elektroden aufweisen. Dies wird nachfolgend noch im Detail erläutert.

Zur Verabreichung des Lokalanästhetikums oder zur Neurostimulation wird der Katheter 1 auf eine dem Fachmann bekannte Weise angelegt und so weit distal vorgeschoben, bis die Katheterspitze 3 in unmittelbarer Nähe zu einem zu betäubenden Nerv platziert ist. Das Anlegen des Katheters 1 erfolgt vorzugsweise unter Zuhilfenahme einer Einführhilfe, was nachfolgend noch näher erläutert wird.

Kommt es während der Schmerztherapie zu einer unbeabsichtigten proximalen Bewegung der Katheterspitze 3, einer sogenannten (proximalen) Dislokation, wird der zu betäubende Nerv, im Falle der Verabreichung eines Lokalanästhetikums, nicht mehr ausreichend von dem Lokalanästhetikum umspült, oder, im Falle der elektrischen Neurostimulation, nur noch suboptimal stimuliert. Hierdurch kann die Wirksamkeit der Schmerztherapie beeinträchtigt werden. Um dem entgegenzuwirken, weist der Katheterschaft 2 einen Vorspannabschnitt 23 auf.

Der Vorspannabschnitt 23 ist zwischen einem Vorspannzustand V und einem Ausgleichszustand A elastisch deformierbar. In dem Vorspannzustand V ist der Vorspannabschnitt 23 axial elastisch gestaucht und weist eine erste Länge L1 auf. In dem Ausgleichszustand A ist der Vorspannabschnitt 23 nicht oder weniger stark axial gestaucht und weist eine zweite Länge L2 auf. Die zweite Länge L2 ist größer als die erste Länge L1. Dadurch kann bei einer proximalen Dislokation des Katheteransatzes 4 eine proximale Dislokation der Katheterspitze 3 vermieden oder zumindest verringert werden.

Wenn der Katheter 1 angelegt und die Katheterspitze 3 in unmittelbarer Nähe zu dem zu betäubenden Nerv platziert ist, spannt der Vorspannabschnitt 23 das distale Ende 22 in distaler Richtung und das proximalen Ende 21 in proximaler Richtung vor. Hierdurch drückt das distale Ende 22 mit der Katheterspitze 3 distal mit einer Vorspannkraft F gegen das den zu betäubenden Nerv umgebende Körpergewebe. Entsprechend übt das die Katheterspitze 3 umgebende Körpergewebe eine der Vorspannkraft F entgegengerichtete und nicht näher dargestellte gleich große Druckkraft, d.h. eine axiale Reaktionskraft, auf die Katheterspitze 3 und über das distale Ende 22 auf den Vorspannabschnitt 23 aus. Der Vorspannabschnitt 23 wird dadurch in dem Vorspannzustand V gehalten und behält seine erste Länge L1 bei. Erfolgt in diesem Zustand eine proximale Dislokation des Katheteransatzes 4, bewegt sich die Katheterspitze 3 proximal um eine infinitesimale Weglänge. Die axiale Druckkraft, die das Körpergewebe auf die Katheterspitze 3 ausübt und der Vorspannkraft F entgegenwirkt, verringert sich dadurch oder wird sogar Null. Infolgedessen streckt sich der Vorspannabschnitt 23, bis die Katheterspitze 3 wieder gegen das distal liegende Körpergewebe anliegt und wieder ein Kräftegleichgewicht zwischen der Vorspannkraft F und der durch das Körpergewebe auf die Katheterspitze 3 ausgeübten Druckkraft herrscht. Auf diese Weise kann durch den Vorspannabschnitt 23 bei einer proximalen Dislokation des Katheteransatzes 4 eine proximale Dislokation der Katheterspitze 3 vermieden werden.

Fig. 2 zeigt den Vorspannabschnitt 23 im Ausgleichszustand A, in dem der Vorspannabschnitt 23 die zweite Länge L2 aufweist. Die zweite Länge L2 ist größer als die erste Länge L1. Die Differenz zwischen der zweiten Länge L2 und der ersten Länge L1 entspricht in etwa oder genau der proximalen Dislokation des Katheteransatzes 4. In dem Ausgleichszustand A gleicht der Vorspannabschnitt 23 durch die Veränderung seiner Länge die proximale Dislokation des Katheteransatzes 4 aus. Bei dem gezeigten Ausführungsbeispiel entspricht der Ausgleichszustand A einem Ruhezustand des Vorspannabschnitts 23, in dem keine äußeren axialen Kräfte auf den Vorspannabschnitt 23 wirken. Entsprechend übt der Vorspannabschnitt 23 selbst keine Vorspannkraft F auf das distale Ende 22 aus. In dem in Fig. 2 gezeigten Ausführungsbeispiel kann sich der Vorspannabschnitt 23 ohne Einwirkung einer externen Kraft, d.h. von selbst, nicht weiter strecken. Bei anderen Ausführungsformen übt der Vorspannabschnitt 23 auch in dem Ausgleichszustand A auf das umgebende Körpergewebe eine Vorspannkraft F aus, die geringer ist als die Vorspannkraft F im Vorspannzustand V. Bei einer weiteren Verringerung der auf die Katheterspitze 3 axial wirkenden Druckkraft durch das Körpergewebe könnte sich der Vorspannabschnitt 23 noch weiter strecken. Entsprechend ist die zweite Länge L2 des Vorspannabschnitts 23 im Ausgleichszustand A geringer als eine maximale Länge des Vorspannabschnitts 23. Bei der in den Fig. 1 und 2 gezeigten Ausführungsform ist der Vorspannabschnitt 23 lediglich über einen Teil einer Gesamtlänge des Katheterschafts 2 längserstreckt. Dabei sind die Anordnung des Vorspannabschnitts 23 und dessen Anteil an der Gesamtlänge als exemplarisch zu verstehen.

Vorliegend ist der Vorspannabschnitt 23 im Bereich des distalen Endes 22 des Katheterschafts 2 angeordnet. Der Katheterschaft 2 weist weiter einen proximalen Schaftabschnitt 24 und einen distalen Schaftabschnitt 25 auf. Der proximale Schaftabschnitt 24 ist axial zwischen dem proximalen Ende 21 und dem Vorspannabschnitt 23 angeordnet. Der proximale Schaftabschnitt 24 umfasst vorliegend das proximale Ende 21 des Katheterschafts 2 und ist einends fest mit dem Katheteransatz 4 verbunden. Andernends ist der proximale Schaftabschnitt 24 fest mit dem Vorspannabschnitt 23 verbunden. Der distale Schaftabschnitt 25 ist axial zwischen der Katheterspitze 3 und dem Vorspannabschnitt 23 angeordnet. Der distale Schaftabschnitt 25 umfasst vorliegend das distale Ende 22 und ist einends fest mit der Katheterspitze 3 verbunden. Andernends ist der distale Schaftabschnitt 25 fest mit dem Vorspannabschnitt 23 verbunden.

Bei einer in den Figuren nicht gezeigten Ausführungsform ist kein distaler Schaftabschnitt 25 vorhanden und der Vorspannabschnitt 23 ist stattdessen unmittelbar mit der Katheterspitze 3 verbunden.

Der proximale Schaftabschnitt 24 und der distale Schaftabschnitt 25 sind weniger leicht axial deformierbar als der Vorspannabschnitt 23. Mit anderen Worten ausgedrückt, weist der Vorspannabschnitt 23 eine größere Drucknachgiebigkeit (geringere Federsteifigkeit) auf als der proximale Schaftabschnitt 24 und der distale Schaftabschnitt 25. Umgekehrt ausgedrückt weisen der proximale Schaftabschnitt 24 und der distale Schaftabschnitt 25 eine geringere Drucknachgiebigkeit (größere Federsteifigkeit) auf als der Vorspannabschnitt 23. Die unterschiedliche Gestaltung im Hinblick auf die elastische Deformierbarkeit kann über eine entsprechend unterschiedliche Materialwahl und/oder Gestaltgebung und/oder durch Vorsehen einer Feder und/oder eines komprimierbaren Gas- oder Flüssigkeitsbehälters oder dergleichen erreicht werden.

Bei der in den Fig. 1 und 2 gezeigten Ausführungsform weist der Vorspannabschnitt 23 eine Drucknachgiebigkeit Z1 auf. Abseits des Vorspannabschnitts 23, d.h. im Bereich des proximalen Schaftabschnitts 24 und des distalen Schaftabschnitts 25, weist der Katheterschaft 2 eine geringere Drucknachgiebigkeit Z2 auf. Die beiden Drucknachgiebigkeiten Z1, Z2 können auch als erste Drucknachgiebigkeit Z1 und zweite Drucknachgiebigkeit Z2 bezeichnet werden. Mit anderen Worten ausgedrückt, ist aufgrund der unterschiedlichen Materialien der Vorspannabschnitt 23 elastisch stauchbar, während abseits des Vorspannabschnitts 23 der Katheterschaft 2 starr oder zumindest vergleichsweise steif oder starr ist.

Die zweite Drucknachgiebigkeit Z2 des Katheterschafts 2 abseits des Vorspannabschnitts 23 beträgt vorliegend lediglich 2 % der ersten Drucknachgiebigkeit Z1. Bei in den Figuren nicht gezeigten Ausführungsformen beträgt die zweite Drucknachgiebigkeit maximal 20 %, bevorzugt maximal 5 %, der ersten Drucknachgiebigkeit Z1.

Bei der Ausführungsform nach den Fig. 1 und 2 ist der Vorspannabschnitt 23 aus einem Material M1 gefertigt. Abseits des Vorspannabschnitts 23, d.h. im Bereich des proximalen Schaftabschnitts 24 und des distalen Schaftabschnitts 25, ist der Katheterschaft 2 aus einem unterschiedlichen Material M2 gefertigt. Die beiden Materialien M1, M2 können auch als erstes Material M1 und zweites Material M2 bezeichnet werden.

Bei beiden Materialien M1, M2 handelt es sich vorliegend um für medizinische Anwendungen geeignete Kunststoffwerkstoffe. Die Materialien M1, M2 sind wenigstens im Hinblick auf ihre Drucknachgiebigkeit und/oder Federsteifigkeiten unterschiedlich.

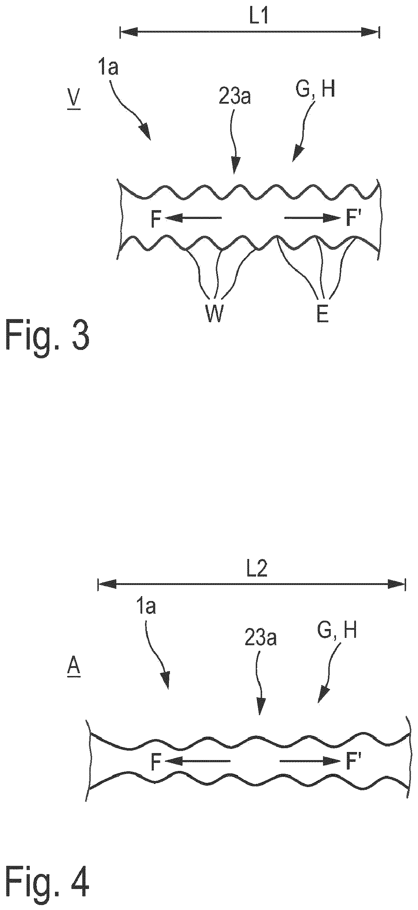

Bei einer Ausführungsform nach den Fig. 1 und 2 wird die elastische Deformierbarkeit des Vorspannabschnitts 23 folglich über eine entsprechende Materialwahl erreicht. Alternativ oder zusätzlich kann der Vorspannabschnitt 23 eine elastisch stauchbare Gestaltgebung aufweisen. Eine solche elastisch stauchbare Gestaltgebung ist exemplarisch in den Fig. 3 und 4 gezeigt.

Die Fig. 3 und 4 zeigen eine Ausführungsform eines Katheters 1a, bei welcher der Vorspannabschnitt 23a eine elastisch stauchbare Gestaltgebung G aufweist. Im Speziellen ist der Vorspannabschnitt 23a nach Art einer Ziehharmonika gestaltet und weist insoweit eine ziehharmonikaartige Gestaltung H auf. Der Katheterschaft 2 ist folglich im Bereich des Vorspannabschnitts 23 mit radialen Ausbuchtungen W und radialen Einbuchtungen E versehen. Die Ausbuchtungen W und die Einbuchtungen E wechseln einander in axialer Richtung ab und bilden die besagte ziehharmonikaartige Gestaltung H.

Fig. 3 zeigt den Vorspannabschnitt 23a im Vorspannzustand V. Fig. 4 zeigt den Vorspannabschnitt 23a im Ausgleichszustand A. Im Vorspannzustand V übt der Vorspannabschnitt 23a die distale Vorspannkraft F in Richtung des distal der Katheterspitze gelegenen Körpergewebes und eine entgegengerichtete, betragsmäßig gleich große und von der distalen Vorspannkraft F weg weisende proximale Vorspannkraft F' in Richtung des Katheteransatzes aus. Bei einer proximalen Dislokation wirkt der distalen Vorspannkraft F keine gleich große proximale Druckkraft mehr entgegen, so dass sich die ziehharmonikaartige Gestaltung H axial so weit streckt, bis die durch das Körpergewebe auf die Katheterspitze 3 ausgeübte proximale Druckkraft die distale Vorspannkraft F wieder ausgleicht. Die Vorspannkraft F ist im Ausgleichszustand A gemäß Fig. 4 geringer als im Vorspannzustand V gemäß Fig. 3, was bildlich durch einen kürzeren Kraftpfeil veranschaulicht ist. In gleicher Weise verringert sich auch die proximale Vorspannkraft F' zwischen dem Vorspannzustand V und dem Ausgleichszustand A. Im Ausgleichszustand A hat die ziehharmonikaartige Gestaltung H eine proximale Dislokation mit einer Länge ausgeglichen, die der Differenz zwischen der zweiten Länge L2 der ziehharmonikaartigen Gestaltung H im Ausgleichszustand A und der ersten Länge L1 der ziehharmonikaartigen Gestaltung H im Vorspannzustand V entspricht.

Es versteht sich, dass die in den Fig. 3 und 4 gezeigte Anzahl, Formgebung und Dimensionierung der Ausbuchtungen W und der Einbuchtungen E als rein exemplarisch zu verstehen sind.

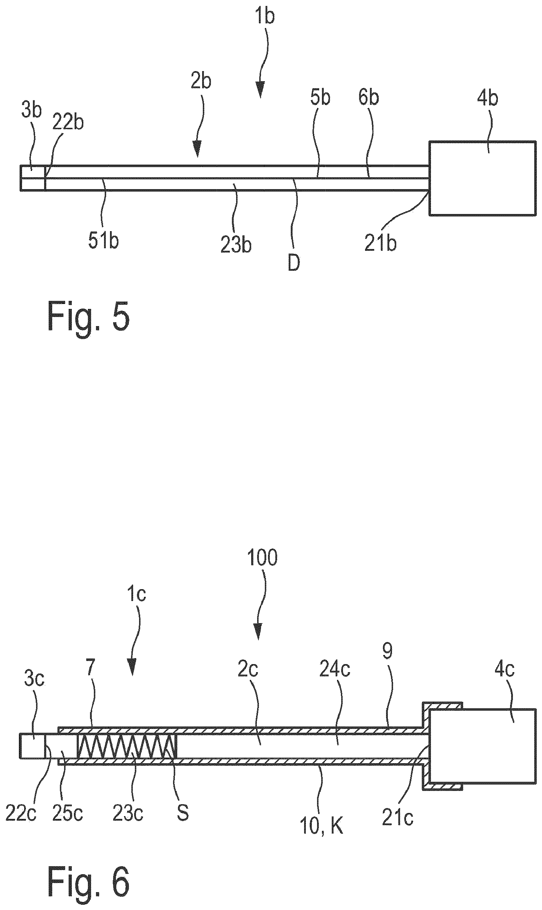

Die Fig. 5 bis 7 zeigen weitere Ausführungsformen erfindungsgemäßer Katheter 1b, 1c, 1d. Zur Vermeidung von Wiederholungen wird nachfolgend in erster Linie auf wesentliche Unterschiede der Katheter 1b, 1c, 1d gegenüber dem Katheter 1 und dem Katheter 1a nach den Fig. 1 bis 4 eingegangen. Funktionsgleiche Bauteile und/oder Abschnitte der Katheter 1b, 1c, 1d sind mit identischen Bezugszeichenziffern unter Hinzufügung von Kleinbuchstaben versehen. Sofern nichts anderes beschrieben ist, gilt das zu den Kathetern 1, 1a nach den Fig. 1 bis 4 Offenbarte, mutatis mutandis, auch für die Katheter 1b, 1c, 1d nach den Fig. 5 bis 7.

Bei dem Katheter 1b nach Fig. 5 ist der Vorspannabschnitt 23b über eine gesamte Länge des Katheterschafts 2b erstreckt. Hierdurch kann im Vergleich zu dem Katheter 1 nach den Fig. 1 und 2 eine einfachere Herstellung erreicht werden. Zudem kann der Vorspannabschnitt 23 über eine größere Länge elastisch gestaucht werden, wodurch größere proximale Dislokationen des Katheteransatzes 4 ausgeglichen werden können.

Der Katheter 1b weist eine Längenbegrenzungseinrichtung 5b auf. Die Längenbegrenzungseinrichtung 5b ist zur Begrenzung der Länge oder einer Längenzunahme des Vorspannabschnitts 23b eingerichtet. Die Längenbegrenzungseinrichtung 5b kann bei unterschiedlichen Ausgestaltungen unterschiedlich gestaltet sein.

Bei der Ausführungsform der Fig. 5 weist die Längenbegrenzungseinrichtung 5b ein Zugelement 51b auf, das den Vorspannabschnitt 23b unter Ausbildung einer Kraftentlastung überbrückt. Sobald der Vorspannabschnitt 23b eine maximal zulässige zweite Länge L2 im Ausgleichszustand A erreicht, wirkt die Längenbegrenzungseinrichtung 5b als Kraftentlastung D und bewirkt einen Katheterschaft-internen Kraftausgleich der Vorspannkraft F, so dass der Vorspannabschnitt 23b sich nicht weiter strecken kann. In diesem Zustand kann eine proximale Dislokation des Katheteransatzes 4b unmittelbar zu einer proximalen Dislokation der Katheterspitze 3b führen. Beispielsweise kann bei mittels des Zugelements 51b kraftentlastetem Vorspannabschnitt 23b ein Entfernen des Katheters 1b aus dem Körpergewebe erleichtert sein.

Da bei der in Fig. 5 gezeigten Ausführungsform der Vorspannabschnitt 23b sich über die gesamte Länge des Katheterschafts 2b erstreckt, erstreckt sich entsprechend auch das Zugelement 51b über die gesamte Länge des Katheterschafts 2b. Bei anderen nicht gezeigten Ausführungsformen, bei denen sich der Vorspannabschnitt nur über einen Teil der Gesamtlänge des Katheterschafts erstreckt, kann sich auch das Zugelement nur über die Länge des Vorspannabschnitts erstrecken oder alternativ über die gesamte Länge des Katheterschafts.

Bei der in Fig. 5 gezeigten Ausführungsform ist zudem eine Halteeinrichtung 6b vorgesehen, die dazu eingerichtet ist, den Vorspannabschnitt 23b im Vorspannzustand V gegen eine Derformation zu sichern. Die Halteeinrichtung 6b ist aktivierbar. Bei einer Aktivierung gibt die Halteeinrichtung 6b eine Deformation des Vorspannabschnitts 23b frei. Das bedeutet, dass erst nach Aktivierung der Halteeinrichtung 6b eine Deformation des Vorspannabschnitts 23b möglich ist. Bei manchen Ausführungsformen kann die Halteeinrichtung 6b derart gestaltet sein, dass sie vor einer Aktivierung eine Stauchung des Vorspannabschnitts 23b zulässt, während sie eine Streckung des Vorspannabschnitts 23b verhindert. Bei anderen Ausführungsformen kann die Halteeinrichtung 6b derart gestaltet sein, dass sie vor einer Aktivierung weder eine Stauchung noch eine Streckung des Vorspannabschnitts 23b zulässt.

Die Halteeinrichtung 6b kann ein Anlegen des Katheters 1b und ein distales Vorschieben der Katheterspitze 3b in unmittelbare Nähe des zu betäubenden Nervs erleichtern, indem beim Vorschieben die Halteeinrichtung 6b nicht aktiviert ist, so dass der Katheter 1b mit unveränderter oder unveränderbarer Länge distal vorgeschoben werden kann. Nach Platzierung der Katheterspitze 3b in unmittelbarer Nähe des zu betäubenden Nervs erfolgt die Aktivierung der Halteeinrichtung 6b, so dass der Vorspannabschnitt 23b eine proximale Dislokation ausgleichen kann.

Bei der gezeigten Ausführungsform fungiert das Zugelement 51b auch als Halteeinrichtung 6b und erfüllt demnach sowohl die Längenbegrenzungsfunktion als auch die Freigabefunktion.

Bei der in Fig. 6 gezeigten Ausführungsform erstreckt sich der Vorspannabschnitt 23c über nur einen Teil der gesamten Länge des Katheterschafts 2c. Der Vorspannabschnitt 23c weist eine Feder S auf. Speziell ist die Feder S als eine Schraubendruckfeder gestaltet.

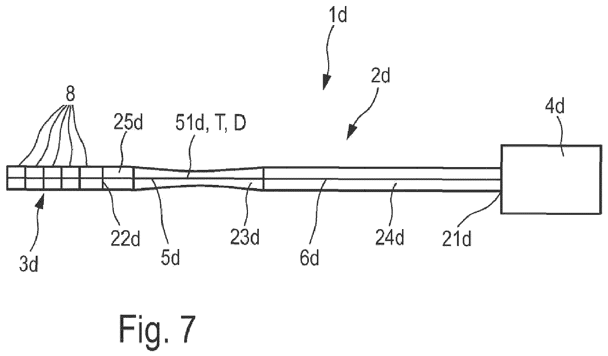

Der in Fig. 7 gezeigte Katheter 1d ist als Stimulationskatheter zur elektrischen Neurostimulation ausgebildet. Zu diesem Zweck weist der Katheter 1d an der Katheterspitze 3d mehrere Stimulationselektroden 8 auf, speziell sind fünf Stimulationselektroden 8 gezeigt. Über die Stimulationselektroden 8 kann der Katheter 1d elektrische Stromimpulse an das die Katheterspitze 3d umgebende Körpergewebe abgeben. Die Stimulationselektroden 8 sind über einen elektrisch leitenden Draht T mit dem Katheteransatz 4d verbunden. Der Draht T erstreckt sich somit von der Katheterspitze 3d über die gesamte Länge des Katheterschafts 2d bis zum Katheteransatz 4d. Insbesondere erstreckt sich der Draht T über die gesamte Länge des Vorspannabschnitts 23d, an den distal ein distaler Schaftabschnitt 25d und proximal ein proximaler Schaftabschnitt 24d grenzt. Der Draht T ist zur Übertragung von Stromimpulsen zur Neurostimulation des umgebenden Körpergewebes eingerichtet. Darüber hinaus fungiert der Draht T als Zugelement 51d, das den Vorspannabschnitt 23d unter Ausbildung einer Kraftentlastung überbrücken kann. Zudem fungiert der elektrisch leitende Draht T als Längenbegrenzungseinrichtung 5d zur Begrenzung der zweiten Länge L2 des Vorspannabschnitts 23d. Um die Funktion der Längenbegrenzungseinrichtung 5d und der Halteeinrichtung 6d zu erfüllen, kann der elektrisch leitende Draht T mit dem Vorspannabschnitt 23d an den Kontaktstellen jeweils mit dem proximalen Schaftabschnitt 24d und dem distalen Schaftabschnitt 25d verbunden sein.

Bei nicht gezeigten Ausführungsformen kann der Katheter sowohl zur Abgabe eines Lokalanästhetikums als auch zur elektrischen Neurostimulation ausgebildet sein und dementsprechend ein Katheterlumen und wenigstens einen Katheterauslass sowie wenigstens eine Stimulationselektrode aufweisen.

Fig. 6 zeigt eine Katheteranordnung 100 mit dem bereits vorhergehend beschriebenen Katheter 1c und mit einer Einführhilfe 10. Die Einführhilfe 10 ist vorliegend als Kapillar K gestaltet. Die Einführhilfe 10 weist ein Einführlumen 9 auf, das zur Aufnahme des Katheterschafts 2c eingerichtet ist. In der in Fig. 6 gezeigten Konfiguration ist der Katheterschaft 2c über ein nicht näher bezeichnetes proximales Ende der Einführhilfe 10 in das Einführlumen 9 eingeschoben, wobei ein Teil des distalen Endes 22c des Katheterschafts 2c mitsamt der Katheterspitze 3c aus dem Einführlumen 9 der Einführhilfe 10 distal hervorragt. Der Vorspannabschnitt 23c ist vollständig, d.h. über seine gesamte Länge hinweg, in dem Einführlumen 9 angeordnet. Der Katheteransatz 4c und das proximale Ende der Einführhilfe 10 sind vorzugsweise zum Ausbilden einer lösbaren Verbindung eingerichtet. Die lösbare Verbindung kann eine Schraub-, Luer- oder sonstige für den vorliegenden Zweck geeignete Verbindung sein.

Die Einführhilfe 10 erfüllt bei der in Fig. 6 gezeigten Ausführungsform eine Mehrfachfunktion. Zum einen unterstützt sie ein Anlegen des Katheters 1c. Zum anderen fungiert die Einführhilfe 10 als eine Stützstruktur 7, die den Vorspannabschnitt 23c gegen eine Knickbewegung in einer Richtung quer zur Längserstreckung des Katheterschafts 2c sichert. Dadurch, dass die Einführhilfe 10 die Feder S über die gesamte Länge hinweg und über den gesamten Umfang umgibt, kann die druckbelastete Feder S nicht zur Seite ausweichen oder ausknicken. Das gewährleistet eine zuverlässige Funktion der Feder S. Außerdem kann die Einführhilfe 10 als Schutz des Vorspannabschnitts 23c vor äußeren Einflüssen dienen und dadurch beispielsweise einen Transport der Katheteranordnung 100 erleichtern.

Zusätzlich ist denkbar, dass über das Einführlumen ein Lokalanästhetikum abgegeben wird und über einen in dem Einführlumen angeordneten Stimulationskatheter Stromimpulse an das umgebende Körpergewebe abgegeben werden. Das Lokalanästhetikum kann in diesem Fall beispielsweise durch den Spalt zwischen der Einführhilfe und dem Stimulationskatheter zum distalen Ende der Einführhilfe geleitet werden.

Bei einer alternativen, nicht gezeigten Ausführungsform ist eine separate Stützstruktur vorgesehen, die den Vorspannabschnitt gegen eine Knickbewegung sichert. Die Stützstruktur kann beispielsweise an dem distalen Schaftabschnitt und/oder an dem proximalen Schaftabschnitt und/oder an Vorspannabschnitt befestigt sein.

Es versteht sich, dass einzelne Merkmale der Katheter 1, 1a, 1b, 1c, 1d unter Ausbildung weiterer erfindungsgemäßer Ausführungsformen miteinander kombiniert werden können.

## Patentansprüche

1. Katheter (1, 1a, 1b, 1c, 1d) zur Verwendung bei einer Schmerztherapie, aufweisend:
einen Katheterschaft (2, 2b, 2c, 2d), der zwischen einem proximalen Ende (21, 21b, 21c, 21d) und einem distalen Ende (22, 22b, 22c, 22d) längserstreckt ist,
eine Katheterspitze (3, 3b, 3c, 3d), die an dem distalen Ende (22, 22b, 22c, 22d) angeordnet ist, und
einen Katheteransatz (4, 4b, 4c, 4d), der an dem proximalen Ende (21, 21b, 21c, 21d) angeordnet ist,
**dadurch gekennzeichnet, dass** der Katheterschaft (2, 2b, 2c, 2d) einen Vorspannabschnitt (23, 23a, 23b, 23c, 23d) aufweist, der zwischen einem Vorspannzustand (V) und einem Ausgleichszustand (A) elastisch deformierbar ist, wobei der Vorspannabschnitt (23, 23a, 23b, 23c, 23d) in dem Vorspannzustand (V) axial elastisch gestaucht ist und eine erste Länge (L1) aufweist, und wobei der Vorspannabschnitt (23, 23a, 23b, 23c, 23d) in dem Ausgleichszustand (A) nicht oder weniger stark axial gestaucht ist und ein größere zweite Länge (L2) aufweist, wodurch bei einer proximalen Dislokation des Katheteransatzes (4, 4b, 4c, 4d) eine proximale Dislokation der Katheterspitze (3, 3b, 3c, 3d) vermieden ist.

2. Katheter (1, 1a, 1b, 1c, 1d) nach Anspruch 1, **dadurch gekennzeichnet, dass** der Vorspannabschnitt (23, 23a, 23b, 23c, 23d) eine elastisch stauchbare Gestaltgebung (G) aufweist und/oder aus einem elastisch stauchbaren Material (M1) gefertigt ist und/oder eine Feder (S) aufweist.

3. Katheter (1, 1a, 1b, 1c, 1d) nach Anspruch 2, **dadurch gekennzeichnet, dass** die elastisch stauchbare Gestaltgebung (G) eine ziehharmonikaartige Gestaltung (H) ist.

4. Katheter (1, 1a, 1b, 1c, 1d) nach einem der vorhergehenden Ansprüche, **dadurch gekennzeichnet, dass** der Vorspannabschnitt (V) über eine gesamte Länge des Katheterschafts (2, 2b, 2c, 2d) längserstreckt ist.

5. Katheter (1, 1a, 1b, 1c, 1d) nach einem der Ansprüche 1 bis 3, **dadurch gekennzeichnet, dass** der Vorspannabschnitt (23, 23a, 23b, 23c, 23d) über lediglich einen Teil der Gesamtlänge des Katheterschafts (2, 2b, 2c, 2d) längserstreckt ist und der Katheterschaft (2, 2b, 2c, 2d) abseits des Vorspannabschnitts (23, 23a, 23b, 23c, 23d) eine Drucknachgiebigkeit (Z2) aufweist, die geringer ist als eine Drucknachgiebigkeit (Z1) des Vorspannabschnitts (23, 23a, 23b, 23c, 23d).

6. Katheter (1, 1a, 1b, 1c, 1d) nach Anspruch 5, **dadurch gekennzeichnet, dass** der Katheterschaft (2, 2b, 2c, 2d) abseits des Vorspannabschnitts (23, 23a, 23b, 23c, 23d) eine unterschiedliche Gestaltgebung aufweist und/oder aus einem unterschiedlichen Material (M2) gefertigt ist.

7. Katheter (1, 1a, 1b, 1c, 1d) nach einem der vorhergehenden Ansprüche, **gekennzeichnet durch** eine Längenbegrenzungseinrichtung (5), die zur Begrenzung der zweiten Länge (L2) des Vorspannabschnitts (23, 23a, 23b, 23c, 23d) eingerichtet ist.

8. Katheter (1, 1a, 1b, 1c, 1d) nach Anspruch 7, **dadurch gekennzeichnet, dass** die Längenbegrenzungseinrichtung (5b, 5d) ein längserstrecktes Zugelement (51b, 51d) aufweist, das den Vorspannabschnitt (23, 23a, 23b, 23c, 23d) unter Ausbildung einer Kraftentlastung (D) überbrückt.

9. Katheter (1, 1a, 1b, 1c, 1d) nach Anspruch 8, **dadurch gekennzeichnet, dass** das längserstreckte Zugelement (51b, 51d) einen elektrisch leitfähigen Draht (T) aufweist, der zur Übertragung und/oder Abgabe von Stromimpulsen zur Neurostimulation eingerichtet ist.

10. Katheter (1, 1a, 1b, 1c, 1d) nach einem der vorhergehenden Ansprüche, **gekennzeichnet durch** eine Halteeinrichtung (6b, 6d), die dazu eingerichtet ist, den Vorspannabschnitt (23, 23a, 23b, 23c, 23d) im Vorspannzustand (V) gegen eine Deformation zu sichern und bei einer Aktivierung eine Deformation des Vorspannabschnitts (V) freizugeben.

11. Katheter (1, 1a, 1b, 1c, 1d) nach einem der vorhergehenden Ansprüche, **gekennzeichnet durch** eine Stützstruktur (7), die den Vorspannabschnitt (23, 23a, 23b, 23c, 23d) gegen eine Knickbewegung in einer Richtung quer zur Längserstreckung sichert.

12. Katheter (1, 1a, 1b, 1c, 1d) nach einem der vorhergehenden Ansprüche, **dadurch gekennzeichnet, dass** der Katheterschaft (2, 2b, 2c, 2d) ein Katheterlumen zur Verabreichung eines Lokalanästhetikums aufweist.

13. Katheteranordnung (100) mit einem Katheter (1, 1a, 1b, 1c, 1d) nach einem der vorhergehenden Ansprüche und mit einer Einführhilfe (10), die zwischen einem distalen Ende und einem proximalen Ende längserstreckt ist und ein Einführlumen (9) zum Einführen des Katheterschafts (2, 2b, 2c, 2d) aufweist.

14. Katheteranordnung (100) nach Anspruch 13, **dadurch gekennzeichnet, dass** bei in das Einführlumen (9) eingeführtem Katheter (1, 1a, 1b, 1c, 1d) die Einführhilfe (10) als Stützstruktur (7) für den Vorspannabschnitt (23, 23a, 23b, 23c, 23d) fungiert.

## Claims

1. Catheter (1, 1a, 1b, 1c, 1d) for use in pain therapy, comprising:
a catheter shaft (2, 2b, 2c, 2d), which extends between a proximal end (21, 21b, 21c, 21d) and a distal end (22, 22b, 22c, 22d),
a catheter tip (3, 3b, 3c, 3d), which is arranged at the distal end (22, 22b, 22c, 22d), and
a catheter hub (4, 4b, 4c, 4d), which is arranged at the proximal end (21, 21b, 21c, 21d),
**characterized in that** the catheter shaft (2, 2b, 2c, 2d) has a pretensioning section (23, 23a, 23b, 23c, 23d), which is elastically deformable between a pretensioning state (V) and a compensating state (A), wherein the pretensioning section (23, 23a, 23b, 23c, 23d) is axially elastically compressed in the pretensioning state (V) and has a first length (L1), and wherein, in the compensating state (A), the pretensioning section (23, 23a, 23b, 23c, 23d) is not axially compressed or is less axially compressed and has a greater, second length (L2), as a result of which, in the event of a proximal dislocation of the catheter hub (4, 4b, 4c, 4d), a proximal dislocation of the catheter tip (3, 3b, 3c, 3d) is avoided.

2. Catheter (1, 1a, 1b, 1c, 1d) according to Claim 1, **characterized in that** the pretensioning section (23, 23a, 23b, 23c, 23d) has an elastically compressible configuration (G) and/or is produced from an elastically compressible material (M1) and/or has a spring (S).

3. Catheter (1, 1a, 1b, 1c, 1d) according to Claim 2, **characterized in that** the elastically compressible configuration (G) is an accordion-like configuration (H).

4. Catheter (1, 1a, 1b, 1c, 1d) according to any one of the preceding claims, **characterized in that** the pretensioning section (23, 23a, 23b, 23c, 23d) extends over an entire length of the catheter shaft (2, 2b, 2c, 2d).

5. Catheter (1, 1a, 1b, 1c, 1d) according to any one of Claims 1 to 3, **characterized in that** the pretensioning section (23, 23a, 23b, 23c, 23d) extends over only a part of the total length of the catheter shaft (2, 2b, 2c, 2d), and the catheter shaft (2, 2b, 2c, 2d), away from the pretensioning section (23, 23a, 23b, 23c, 23d), has a compressive compliance (Z2) which is less than a compressive compliance (Z1) of the pretensioning section (23, 23a, 23b, 23c, 23d).

6. Catheter (1, 1a, 1b, 1c, 1d) according to Claim 5, **characterized in that** the catheter shaft (2, 2b, 2c, 2d), away from the pretensioning section (23, 23a, 23b, 23c, 23d), has a different configuration and/or is produced from a different material (M2).

7. Catheter (1, 1a, 1b, 1c, 1d) according to any one of the preceding claims, **characterized by** a length-limiting device (5) which is configured to limit the second length (L2) of the pretensioning section (23, 23a, 23b, 23c, 23d).

8. Catheter (1, 1a, 1b, 1c, 1d) according to Claim 7, **characterized in that** the length-limiting device (5b, 5d) has an elongate tension element (51b, 51d) which bridges the pretensioning section (23, 23a, 23b, 23c, 23d) to form a force relief (D).

9. Catheter (1, 1a, 1b, 1c, 1d) according to Claim 8, **characterized in that** the elongate tension element (51b, 51d) has an electrically conductive wire (T), which is configured for transmission and/or delivery of current pulses for neurostimulation.

10. Catheter (1, 1a, 1b, 1c, 1d) according to any one of the preceding claims, **characterized by** a holding device (6b, 6d) which is configured to secure the pretensioning section (23, 23a, 23b, 23c, 23d) against deformation in the pretensioning state (V) and, upon activation, to enable a deformation of the pretensioning section (23, 23a, 23b, 23c, 23d).

11. Catheter (1, 1a, 1b, 1c, 1d) according to any one of the preceding claims, **characterized by** a support structure (7) which secures the pretensioning section (23, 23a, 23b, 23c, 23d) against a buckling movement in a direction transverse to the longitudinal extent.

12. Catheter (1, 1a, 1b, 1c, 1d) according to any one of the preceding claims, **characterized in that** the catheter shaft (2, 2b, 2c, 2d) has a catheter lumen for administering a local anaesthetic.

13. Catheter arrangement (100) having a catheter (1, 1a, 1b, 1c, 1d) according to any one of the preceding claims and having an insertion aid (10) which extends between a distal end and a proximal end and has an insertion lumen (9) for insertion of the catheter shaft (2, 2b, 2c, 2d).

14. Catheter arrangement (100) according to Claim 13, **characterized in that**, with the catheter (1, 1a, 1b, 1c, 1d) inserted into the insertion lumen (9), the insertion aid (10) functions as a support structure (7) for the pretensioning section (23, 23a, 23b, 23c, 23d).

## Revendications

1. Cathéter (1, 1a, 1b, 1c, 1d) destiné à être utilisé lors d'un traitement de la douleur, comportant :
une tige (2, 2b, 2c, 2d) de cathéter, qui s'étend longitudinalement entre une extrémité proximale (21, 21b, 21c, 21d) et une extrémité distale (22, 22b, 22c, 22d),
une pointe (3, 3b, 3c, 3d) de cathéter, qui est disposée sur l'extrémité distale (22, 22b, 22c, 22d), et
un embout (4, 4b, 4c, 4d) de cathéter, qui est disposé sur l'extrémité proximale (21, 21b, 21c, 21d),
**caractérisé en ce que** la tige (2, 2b, 2c, 2d) de cathéter comporte une section de précontrainte (23, 23a, 23b, 23c, 23d) qui peut être déformée élastiquement entre un état de précontrainte (V) et un état de compensation (A), la section de précontrainte (23, 23a, 23b, 23c, 23d) étant comprimée axialement élastiquement dans l'état de précontrainte (V) et présentant une première longueur (L1), et la section de précontrainte (23, 23a, 23b, 23c, 23d) n'étant pas comprimée ou étant moins comprimée axialement élastiquement dans l'état de compensation (A) et présentant une deuxième longueur (L2) plus grande, ce qui permet d'éviter une dislocation proximale de l'embout (4, 4b, 4c, 4d) de cathéter et une dislocation proximale de la pointe (3, 3b, 3c, 3d) de cathéter.

2. Cathéter (1, 1a, 1b, 1c, 1d) selon la revendication 1, **caractérisé en ce que** la section de précontrainte (23, 23a, 23b, 23c, 23d) présente une configuration élastiquement compressible (G) et/ou est produite à partir d'un matériau élastiquement compressible (M1) et/ou comporte un ressort (S).

3. Cathéter (1, 1a, 1b, 1c, 1d) selon la revendication 2, **caractérisé en ce que** la configuration élastiquement compressible (G) est une géométrie en accordéon (H).

4. Cathéter (1, 1a, 1b, 1c, 1d) selon l'une des revendications précédentes, **caractérisé en ce que** la section de précontrainte (V) s'étend longitudinalement sur une longueur totale de la tige (2, 2b, 2c, 2d) de cathéter.

5. Cathéter (1, 1a, 1b, 1c, 1d) selon l'une des revendications 1 à 3, **caractérisé en ce que** la section de précontrainte (23, 23a, 23b, 23c, 23d) s'étend longitudinalement sur seulement une partie de la longueur totale de la tige (2, 2b, 2c, 2d) de cathéter et la tige (2, 2b, 2c, 2d) de cathéter présente, en dehors de la section de précontrainte (23, 23a, 23b, 23c, 23d), une compliance à la pression (Z2) qui est inférieure à une compliance à la pression (Z1) de la section de précontrainte (23, 23a, 23b, 23c, 23d).

6. Cathéter (1, 1a, 1b, 1c, 1d) selon la revendication 5, **caractérisé en ce que** la tige (2, 2b, 2c, 2d) de cathéter présente, en dehors de la section de précontrainte (23, 23a, 23b, 23c, 23d), une configuration différente et/ou est produite à partir d'un matériau différent (M2).

7. Cathéter (1, 1a, 1b, 1c, 1d) selon l'une des revendications précédentes, **caractérisé par** un dispositif de limitation de longueur (5) qui est mis au point pour limiter la deuxième longueur (L2) de la section de précontrainte (23, 23a, 23b, 23c, 23d).

8. Cathéter (1, 1a, 1b, 1c, 1d) selon la revendication 7, **caractérisé en ce que** le dispositif de limitation de longueur (5b, 5d) comporte un élément de traction (51b, 51 d) s'étendant longitudinalement, qui surmonte la section de précontrainte (23, 23a, 23b, 23c, 23d) tout en formant une décharge de force (D).

9. Cathéter (1, 1a, 1b, 1c, 1d) selon la revendication 8, **caractérisé en ce que** l'élément de traction (51b, 51 d) s'étendant longitudinalement comporte un fil électriquement conducteur (T), qui est mis au point pour transmettre et/ou délivrer des impulsions de courant destinées à la neurostimulation.

10. Cathéter (1, 1a, 1b, 1c, 1d) selon l'une des revendications précédentes, **caractérisé par** un dispositif de maintien (6b, 6d) qui est mis au point pour bloquer la section de précontrainte (23, 23a, 23b, 23c), 23d) dans l'état de précontrainte (V) et l'empêcher de se déformer et pour débloquer, lors d'une activation, une déformation de la section de précontrainte (V).

11. Cathéter (1, 1a, 1b, 1c, 1d) selon l'une des revendications précédentes, **caractérisé par** une structure de support (7) qui bloque la section de précontrainte (23, 23a, 23b, 23c, 23d) en empêchant tout mouvement de flambage dans une direction transversalement à l'extension longitudinale.

12. Cathéter (1, 1a, 1b, 1c, 1d) selon l'une des revendications précédentes, **caractérisé en ce que** la tige (2, 2b, 2c, 2d) de cathéter comporte une lumière de cathéter pour administrer un anesthésique local.

13. Ensemble cathéter (100) avec un cathéter (1, 1a, 1b, 1c, 1d) selon l'une des revendications précédentes et avec une aide à l'insertion (10), qui s'étend longitudinalement entre une extrémité distale et une extrémité proximale et comporte une lumière d'insertion (9) pour insérer la tige (2, 2b, 2c, 2d) de cathéter.

14. Ensemble cathéter (100) selon la revendication 13, **caractérisé en ce que** lorsqu'un cathéter (1, 1a, 1b, 1c, 1d) est inséré dans la lumière d'insertion (9), l'aide à l'insertion (10) agit comme structure de support (7) pour la section de précontrainte (23, 23a, 23b, 23c, 23d).
